# FASCICULE DE BREVET EUROPEEN

(11) **EP 2 916 806 B1**
(45) Date de publication et mention de la délivrance du brevet: **26.12.2018**
(21) Numéro de dépôt: 13801638.1
(22) Date de dépôt: 07.11.2013
(51) Int. Cl.: A61K 8/365, A61Q 5/04, A61K 8/41, A61K 8/34, A61K 8/58, A61Q 5/06, A61K 8/73, A61K 8/891

(54) **PROCEDE DE LISSAGE DES CHEVEUX A PARTIR D'UNE COMPOSITION CONTENANT DE L'ACIDE GLYOXYLIQUE ET/OU UN DE SES DERIVES**
VERFAHREN ZUR HAARGLÄTTUNG MIT EINER ZUSAMMENSETZUNG MIT GLYOXYLSÄURE UND/ODER EINEM DERIVAT DAVON
METHOD FOR STRAIGHTENING THE HAIR USING A COMPOSITION CONTAINING GLYOXYLIC ACID AND/OR A DERIVATIVE THEREOF

(30) Priorité: 09.11.2012 FR 1260660
(43) Date de publication de la demande: 16.09.2015
(73) Titulaire: L'Oréal, 75008 Paris (FR)
(72) Inventeur: PAUL, Laurence, F-95320 St Leu la Forêt (FR)
(74) Mandataire: Leray, Noelle
(86) Numéro de dépôt international: PCT/FR2013/052669
(87) Numéro de publication internationale: WO 2014/072645

(56) Documents cités:
- EP-A2- 0 159 628
- WO-A1-2004/012691
- WO-A1-2007/135299
- WO-A1-2012/105985
- WO-A2-2011/104282
- WO-A2-2012/010351
- FR-A1- 2 950 531
- DATABASE mintel [Online] gnpd; octobre 2012 (2012-10), "Luxury Keratin Treatment", XP002703565, Database accession no. 1892615

## Description

La présente invention concerne un procédé particulier de lissage des fibres kératiniques, en particulier des cheveux, à partir d'une composition à l'acide glyoxylique et/ou un de ses dérivés.

Dans le domaine capillaire, les consommateurs souhaitent disposer de compositions permettant d'apporter un changement temporaire à leur chevelure, et ce en visant une bonne tenue de l'effet réalisé. En général, on désire que le changement persiste aux shampooings pendant au minimum 15 jours, voire plus selon la nature dudit changement.

Des traitements existent déjà pour modifier la couleur ou la forme des cheveux ainsi que, dans une certaine mesure, la texture des cheveux. L'un des traitements connus pour modifier la texture des cheveux consiste en l'association de chaleur et d'une composition comprenant du formol. Ce traitement est spécialement efficace pour conférer un meilleur aspect aux cheveux abîmés, et/ou pour traiter les cheveux longs et les cheveux bouclés.

On associe l'action du formol à sa capacité de réticuler les protéines par réaction sur ses sites nucléophiles. La chaleur utilisée peut être celle du fer (pince plate ou fer à friser), dont la température peut atteindre en général 200°C ou plus. Toutefois, on cherche de plus en plus à éviter l'emploi de telles substances, qui peuvent se révéler agressives pour le cheveu et les autres matières kératiniques.

Il a ainsi été proposé, par la demande WO2011/104282, un nouveau procédé pour lisser de manière semi-permanente les cheveux, consistant à appliquer une solution pouvant contenir un alphacétoacide sur le cheveu pendant 15 à 120 minutes, puis à sécher et enfin à lisser au fer, à une température d'environ 200°C, la chevelure. L'alphacétoacide employé est de préférence l'acide glyoxylique.

WO2012/105985 décrit un procédé de lissage des cheveux qui consiste à appliquer une composition qui comprend de l'acide glyoxylique, la composition pouvant contenir de l'alcool cétylique.

Toutefois, on a constaté que l'utilisation de ces alpha cetoacides tels que l'acide glyoxylique, pouvait engendrer quelques limitations importantes; notamment à forte concentration, ils peuvent ne pas être bien tolérés, en particulier quand le cuir chevelu est sensible et/ou irrité. Leur volatilité, amplifiée par l'utilisation de chaleur, par exemple par l'intermédiaire d'un fer à friser ou à lisser, peut également poser problème. Par ailleurs, les formulations cosmétiques à pH acide peuvent altérer les cheveux et/ou en altérer la couleur.

Par ailleurs, ce type de procédé de lissage est souvent associé à différentes étapes de lavage, séchage, conditionnement, mise en forme à la brosse et au sèche-cheveux qui rendent ces procédés fastidieux à mettre en oeuvre, notamment en terme de temps de traitement des cheveux pour obtenir le lissage des cheveux souhaités.
Enfin les procédés de lissage utilisant un fer s'accompagnent souvent de l'émission de fumées, ce qui peut nuire au confort de l'utilisateur de la composition et du coiffeur qui l'applique.
Le but de l'invention est de développer un nouveau procédé de lissage/défrisage des cheveux rapide qui permet de lisser/défriser et/ou de réduire le volume les cheveux de façon efficace et rémanente en limitant la dégradation des cheveux tout en conservant un confort au moment de l'application pour l'utilisateur de la composition mais aussi pour le coiffeur qui l'applique.
Ainsi, un objet de la présente invention est un procédé de lissage/défrisage des fibres kératiniques, en particulier des cheveux, qui comprend (i) l'application sur lesdites fibres d'une composition cosmétique comprenant au moins de l'acide glyoxylique et/ou un de ses dérivés, la composition présentant un pH inférieur ou égal à 4, (ii) un temps de pose de la composition à l'acide glyoxylique et/ou un de ses dérivés d'au moins 10 min, de préférence variant de 10 à 60 min, suivi de (iii) l'application sans rinçage d'une composition comprenant au moins un composé de traitement des cheveux choisi parmi les alcools gras, puis (iv) d'une phase d'application de chaleur comprenant au moins une étape de chauffage à une température d'au moins 150°C.
Avec le procédé de l'invention, on obtient un bon lissage des fibres kératiniques avec une dégradation limitée de ces fibres kératiniques, même lorsque l'application de la composition est suivie par un traitement thermique, notamment au moyen de fer de lissage des cheveux. Elle va en outre permettre d'obtenir un lissage temporaire des cheveux satisfaisant tout en maintenant une rapidité de mise en oeuvre du procédé. On obtient ainsi dans un temps réduit un lissage des cheveux qui limite la dégradation des propriétés physiques des cheveux, tout en réduisant l'effet de frisottis de façon durable. Le confort tant pour l'utilisateur de la composition que pour le coiffeur qui l'applique est satisfaisant
Dans ce qui suit l'expression « au moins un » est équivalente à l'expression « un ou plusieurs ».
De préférence, la composition selon l'invention ne comprend ni agent colorant ni agent réducteur.

Par « agents colorants », on entend selon la présente invention des agents de coloration des fibres kératiniques tels que les colorants directs, les pigments ou les précurseurs de colorant d'oxydation (bases et coupleurs). S'ils sont présents, leur teneur ne dépasse pas 0,001 % en poids par rapport au poids total de la composition. En effet, à une telle teneur, seule la composition serait teintée, c'est-à-dire qu'on n'observerait pas d'effet de coloration des fibres kératiniques.
On rappelle que les précurseurs de colorants d'oxydation, bases d'oxydation et coupleurs, sont des composés peu ou non colorés qui par une réaction de condensation en présence d'un agent oxydant, donnent une espèce colorée. Quant aux colorants directs, ces composés sont colorés et présentent une certaine affinité pour les fibres kératiniques.
Par « agent réducteur », on entend selon la présente invention un agent capable de réduire les liaisons disulfures des cheveux, tel que les composés choisis parmi les thiols, les sulfites alcalins, les hydrures, les phosphines.
Dans la présente invention, l'acide glyoxylique et/ ou ses dérivés peuvent se présenter sous forme libre, sous forme de sels mais aussi sous leurs formes hydrates. Par dérivés d'acide glyoxylique, on entend les esters d'acide glyoxylique, les amides d'acide glyoxylique, les acétals et hémiacétals d'acide glyoxylique. Les esters d'acide glyoxylique sont par exemple obtenus à partir d'acide glyoxylique et d'un mono ou polyalcool.
Par « *mono ou poly alcool* », on entend un composé organique comprenant un groupe hydroxy (monoalcool) ou au moins deux groupes hydroxy (polyalcool ou polyol) ; ledit composé organique hydroxylé pouvant être aliphatique, acyclique, linéaire ou ramifié, ou (hétéro)cyclique, tels que les sucres (mono ou polysaccharides) ou les sucres alcools. Plus particulièrement, le polyalcool comprend de 2 à 100 groupes hydroxy ; et préférentiellement de 2 à 20 groupes hydroxy ; encore plus préférentiellement de 2 à 10 groupes hydroxy, mieux 2 ou 3 groupes hydroxy. De préférence le mono ou polyalcool est choisi parmi le méthanol, l'éthanol, le propanol, l'isopropanol, le butanol, l'hexanol, l'éthylène gllycol, le glycérol, la dihydroxyacétone, le glucose, le sorbitol, le menthol.

On peut en particulier citer à titre d'esters le glyoxylate de méthyle, le glyoxylate d'éthyle, le glyoxylate de glycérol, le glyoxylate de dihydroxyacétone, le diglyoxylate ou triglyoxylate de de glycérol, les mono,di ou tri glyoxylate de sorbitol, les mono,di ou tri glyoxylate de glucose, le glyoxylate de menthyle, leurs acétals, hémiacétals, hydrates .
Les amides d'acide glyoxylique sont par exemple obtenus à partir d'acide glyoxylique et d'une mono ou polyamine organique.

Par « mono ou polyamine », on entend un composé organique comprenant un groupe amino(monoamine) ou au moins deux (et de préférence de 2 à 100, mieux de 2 à 20) groupes amino ; le dit composé organique pouvant être aliphatique, acyclique, linéaire ou ramifié ou (hétéro)cyclique. Par groupe « amino » on entend un groupe amine primaire -NH₂, ou secondaire >NH. De préférence la mono ou polyamine est aliphatique. Cette amine est de préférence choisie parmi la méthylamine, l'éthylamine, la propylamine, l'isopropylamine, la butylamine, l'hexylamine, la monoéthanolamine, la monopropanolamine, la propane-1,2,3-triamine et la diaminoacétone. On peut en particulier citer le *N*-béta-hydroxyéthylamide de l'acide glyoxylique et le *N*-gamma-hydroxypropylamide de l'acide glyoxylique leurs acétals, hémiacétals, et/ou hydrates

Les acétals et hémiacétals d'acide glyoxylique peuvent par exemple obtenus à partir de la réaction d'alcools sur des formes bloquées d'acide glyoxylique puis hydrolyse. Les alcools peuvent être les mêmes que ceux cités pour les esters. Les acétals peuvent également être des acétals cycliques. On peut en particulier citer l'acide diméthoxy acétique, l'acide diéthoxy acétique, l'acide 1,3-dioxane 2 carboxylique, l'acide 1,3-dioxolane 2-carboxylique.

Les sels peuvent être des sels issus de l'interaction des composés de formule (I) avec des acides ou des bases, les acides ou bases pouvant être de nature organique ou minérale.

De préférence les sels sont des sels issus de l'interaction des composés de formule (I) avec des bases. On citera en particuliers les sels de métaux alcalins alcalins ou alcalino terreux et en particulier les sels de sodium.

De préférence l'acide glyoxylique et/ ou ses dérivés sont utilisés sous forme libre (non salifiée) ou sous forme d'hydrate

Ainsi, le procédé de la présente invention est mis en oeuvre sans une étape de déformation permanente à pH basique ni à base de réducteur. Les compositions selon l'invention et notamment celle comprenant l'acide glyoxylique et/ ou ses dérivés peuvent se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type H/E, E/H ou multiple; d'une suspension ou émulsion de consistance molle de type crème (H/E) ou (E/H); d'un gel aqueux ou anhydre, ou de toute autre forme cosmétique.

Selon un mode de réalisation du procédé de l'invention, la composition contenant l'acide glyoxylique et/ ou ses dérivés est aqueuse ou anhydre. Elle est de préférence aqueuse et comprend alors de l'eau à une concentration allant de 5 à 98%, mieux de 5 à 50%, encore mieux de 10 à 40 % en poids par rapport au poids total de la composition.

Selon un mode de réalisation la composition de l'invention se trouve sous forme d'une composition aqueuse comprenant de 0,1 à 20 % d'acide glyoxylique et/ou de ses dérivés, de préférence au moins 3 % d'acide glyoxylique et/ou de ses dérivés, préférentiellement de 3 à 10 % en poids du poids total de la composition.

Le pH de la composition est inférieur à 4, et varie de préférence de 1 à 3, mieux de 1,7 à 3.
La composition contenant l'acide glyoxylique et/ou un de ses dérivés se présente de préférence sous forme de gels, de lotions ou crèmes, de masques, de sérums.
Le procédé de l'invention comprend une étape (ii) de pose de la composition précédemment décrite sur les cheveux. Ce temps de pose varie généralement de 10 à 60 min, de préférence de 20 à 35 min.

A l'étape (iii), et sans rinçage de la composition contenant l'acide glyoxylique et/ou au moins un de ses dérivés, on applique une composition comprenant au moins un composé de traitement des cheveux choisi parmi les alcools gras, de préférence une quantité allant de 0,01 à 10 % en poids, mieux encore entre 0,1 à 8 % en poids, et encore plus préférentiellement de 0,5 à 5% en poids par rapport au poids total de la composition le ou les contenant. L'étape (iii) peut en outre mettre en oeuvre au moins un composé de traitement des cheveux choisi parmi les polymères cationiques et/ou les tensioactifs cationiques. Ces composés peuvent être contenus dans une composition comprenant d'autres ingrédients cosmétiques. Selon ce mode de réalisation la composition appliquée à l'étape (iii) est exempte d'acide glyoxylique et/ou d'un de ses dérivés.
On entend par alcool gras, un alcool aliphatique à longue chaine comprenant de 8 à 40 atomes de carbone, et comprenant au moins un groupe hydroxyle OH. Ces alcools gras ne sont ni oxyalkylénés, ni glycérolés. De préférence, les alcools gras solides sont de structure R-OH avec R désignant un groupe alkyle ou alkényle linéaire ou ramifié, éventuellement substitué par un ou plusieurs groupes hydroxy, comprenant de 8 à 40, mieux de 10 à 30, voire de 12 à 24 atomes, encore mieux de 14 à 22 atomes de carbone.

De préférence les alcools gras de l'invention ne sont pas liquides à température ambiante (25°C), et à pression atmosphérique (1.013. 105 Pa ; 760 mm de Hg).Encore plus préférentiellement ils sont solides dans ces conditions.

De préférence R désignant un groupe alkyle linéaire, éventuellement substitué par un ou plusieurs groupes hydroxy, comprenant de 8 à 40, mieux de 10 à 30, voire de 12 à 24 atomes, encore mieux de 14 à 22 atomes de carbone.

Les alcools gras susceptibles d'être utilisés peuvent être choisis parmi, seul ou en mélange :
- alcool laurique ou laurylique (1-dodécanol) ;
- alcool myristique ou myristylique (1-tétradécanol) ;
- alcool cétylique (1-hexadécanol) ;
- alcool stéarylique (1-octadécanol) ;
- alcool arachidylique (1-eicosanol) ;
- alcool béhénylique (1-docosanol) ;
- alcool lignocérylique (1-tetracosanol) ;
- alcool cérylique (1-hexacosanol) ;
- alcool montanylique (1-octacosanol) ;
- alcool myricylique (1-triacontanol).

Préférentiellement, l'alcool gras est choisi parmi l'alcool cétylique, l'alcool stéarylique, l'alcool béhénylique et leurs mélanges tels que l'alcool cétylstéarylique ou cétéarylique. Les alcools gras peuvent être des mélanges, ce qui signifie que dans un produit commercial peuvent coexister plusieurs espèces notamment de longueur de chaîne différente, sous forme d'un mélange.

Les tensio-actifs cationiques qui peuvent être utilisés à l'étape (iii) sont bien connus en soi, par exemple, on peut citer les sels d'amines grasses primaires, secondaires ou tertiaires, éventuellement polyoxyalkylénées, les sels d'ammonium quaternaire, et leurs mélanges.
A titre de sels d'ammonium quaternaires, on peut notamment citer, par exemple :
- ceux qui présentent la formule générale (I) suivante : 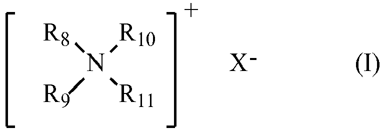 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle, au moins un des radicaux R₈ à R₁₁ comportant de 8 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule (II) suivante : 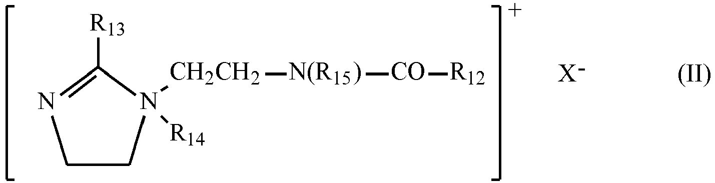 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT® W 75 par la société REWO ;
- les sels de diammonium quaternaire de formule (III) : 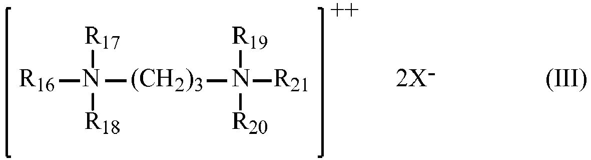 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IV) suivante : 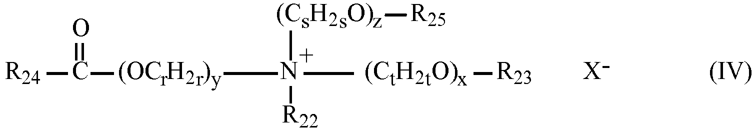 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi :
      - le radical 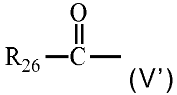
      - les radicaux R₂₇ hydrocarbonés en C₁-C₂₂, linéaires ou ramifiés, saturés ou insaturés,
      - l'atome d'hydrogène,
   R₂₅ est choisi parmi :
      - le radical 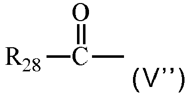
      - les radicaux R₂₉ hydrocarbonés en C1-C6, linéaires ou ramifiés, saturés ou insaturés,
      - l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X- est un anion simple ou complexe, organique ou inorganique ;
   sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.
Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.
On utilise plus particulièrement dans la composition selon l'invention, les sels d'ammonium de formule (IV) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 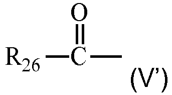
   - les radicaux méthyle, éthyle ou hydrocarbonés en C₁₄-C₂₂,
   - l'atome d'hydrogène ;
- R₂₅ est choisi parmi :
   - le radical 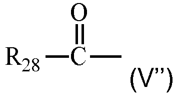
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.
On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire, on préfère en particulier ceux qui répondent à la formule (I) dans laquelle :
R₈ représente un groupe alkyle en C₁₂₋₃₀, de préférence en C₁₄₋₂₂, alcényle en C₁₂₋₃₀, alkyl(C₁₂-C₂₂)amidoalkyle(C₂-C₆), alkyl(C₁₂-C₂₂)acétate, ou un groupe aromatique tel qu'aryle ou alkylaryle en C₆-C₁₂,
R₉ à R₁₁, qui peuvent être identiques ou différents, représentent un groupe alkyle en C₁₋₈, alcényle en C₁₋₈, alcoxy en C₁₋₈, hydroxyalkyle en C₁₋₈, polyoxyalkylène (C₂-C₆) ou alkylamide en C₁₋₈ ; et
X est un anion choisi dans le groupe des halogénures, phosphates, acétates, lactates, alkyl(C₂-C₆)sulfates, alkyl- ou alkylaryl-sulfonates.

A titre d'exemples de tensioactifs cationiques particulièrement préférés, on peut notamment citer d'une part, les sels de tétraalkylammonium, notamment les chlorures de tétraalkylammonium comme, par exemple, les chlorures de dialkyldiméthylammonium ou d'alkyltriméthylammonium dans lesquels le radical alkyle comporte environ de 12 à 30 atomes de carbone, en particulier les chlorures de distéaryldiméthylammonium, de béhényltriméthylammonium, d'arachidyltriméthylammonium, de stéaryltriméthylammonium, de cétyltriméthylammonium, de benzyldiméthylstéarylammonium ou encore, d'autre part, les sels d'alkyl(C₈-C₃₀)amidoalkyl(C₂-C₆)triméthylammonium, notamment le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK le méthosulfate de dipalmitoylethyl hydroxyéthyl méthyl ammonium.

Les tensioactifs cationiques particulièrement préférés sont choisis en particulier parmi le chlorure de béhényltriméthylammonium, le chlorure de cétyl triméthylammonium et le chlorure de palmitylamidopropyltriméthylammonium, le méthosulfate de dipalmitoylethyl hydroxyéthyl méthyl ammonium.

La composition appliquée sur les fibres kératiniques comprend de préférence le ou les tensioactifs cationiques en une quantité allant de 0,01 à 10 % en poids, mieux encore entre 0,1 eà 8 % en poids, et encore plus préférentiellement de 0,5 à 5 % en poids par rapport au poids total de la composition le ou les contenant.

A titre de polymère cationique, on peut citer les polymères comportant des groupements amine primaire, secondaire, tertiaire et/ou quaternaire faisant partie de la chaîne polymère ou directement reliés à celle-ci, et ayant un poids moléculaire compris entre 500 et environ 5 000 000 et de préférence entre 1000 et 3 000 000.

Parmi les polymères cationiques, on peut citer plus particulièrement les polymères du type polyamine, polyaminoamide et polyammonium quaternaire. Ce sont des produits connus. Ils sont par exemple décrits dans les brevets français nos 2 505 348 et 2 542 997.

Parmi ces polymères, on peut citer:
(1) les homopolymères ou copolymères dérivés d'esters ou d'amides acryliques ou méthacryliques et comportant au moins un des motifs de formules suivantes: 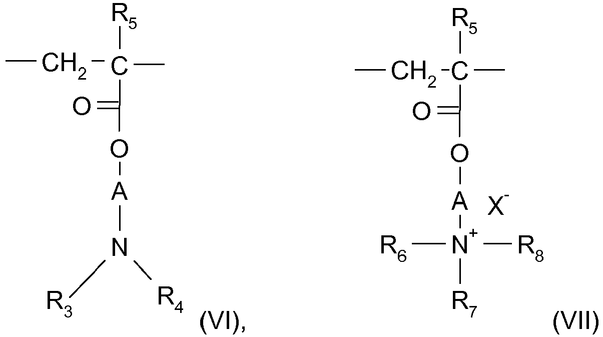 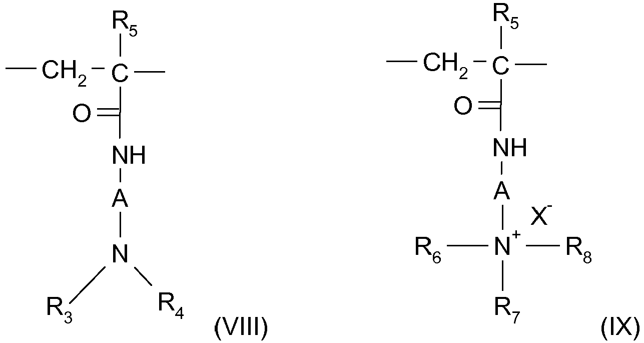 dans lesquelles:
   R3 et R4 , identiques ou différents, représentent hydrogène ou un groupe alkyle ayant de 1 à 6 atomes de carbone et de préférence méthyle ou éthyle;
   R5 , identiques ou différents, désignent un atome d'hydrogène ou un radical CH3;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R6, R7, R8, identiques ou différents, représentent un groupe alkyle ayant de 1 à 18 atomes de carbone ou un radical benzyle et de préférence un groupe alkyle ayant de 1 à 6 atomes de carbone;
   X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylamino-propyle quaternisé tel que le produit vendu sous la dénomination "GAFQUAT HS 100" par la société ISP, et
   - les polymères réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium par exemple en dispersion dans de l'huile minérale ou dans un ester liquide. Ces dispersions sont commercialisées sous les noms de « SALCARE® SC 95 » et « SALCARE® SC 96 » par la Société CIBA.
(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(6) les cyclopolymères d'alkyl diallyl amine ou de dialkyl diallyl ammonium tels que les homopolymères ou copolymères comportant comme constituant principal de la chaîne des motifs répondant aux formules (X) ou (XI): 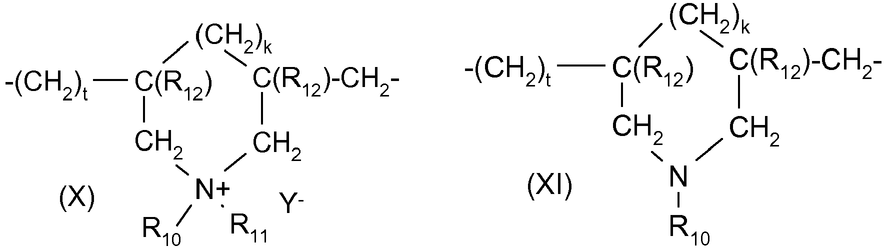 formules dans lesquelles k et t sont égaux à 0 ou 1, la somme k + t étant égale à 1 ; R12 désigne un atome d'hydrogène ou un radical méthyle ; R10 et R11, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R10 et R11 peuvent désigner conjointement avec l'atome d'azote auquel ils sont rattachés, des groupement hétérocycliques, tels que pipéridinyle ou morpholinyle ; Y- est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R10 et R11, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "Merquat 100" par la société NALCO (et ses homologues de faibles masses moléculaires moyenne en poids) et les copolymères de chlorure de diallyldiméthylammonium et d'acrylamide commercialisés sous la dénomination "MERQUAT 550".
(7) le polymère de diammonium quaternaire contenant des motifs récurrents répondant à la formule : 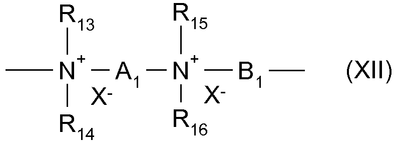 formule (XII) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -C(O)-O-R₁₇-D ou -C(O)-N(H)-R₁₇-D où R₁₇ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻, identique ou différent, désigne un anion dérivé d'un acide minéral ou organique;
   A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement -(CH₂)ₙ-C(O)-D-C(O)-(CH2)p-
   n et p sont des nombres entiers variant de 2 à 20 environ
      dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -N(H)-Y-N(H)-, où Y désigne un radical hydrocarboné linéaire ou ramifié, ou bien le radical bivalent

         -CH₂-CH₂-S-S-CH₂-CH₂- ;
      d) un groupement uréylène de formule : -N(H)-C(O)-N(H)- ;

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 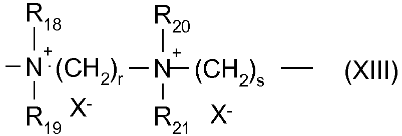 dans laquelle R₁₈, R₁₉, R₂₀ et R₂₁, identiques ou différents, désignent un radical alkyle ou hydroxyalkyle ayant de 1 à 4 atomes de carbone environ, r et s sont des nombres entiers variant de 2 à 20 environ et, X⁻, identique ou différent, est un anion dérivé d'un acide minéral ou organique.
   Un composé de formule (XIII) particulièrement préféré est celui pour lequel R18, R19, R20 et R21, représentent un radical méthyle et r = 3, s = 6 et X = Cl, dénommé Hexadimethrine chloride selon la nomenclature INCI (CTFA).
(8) les polymères de polyammonium quaternaires constitués de motifs de formule (XIV): 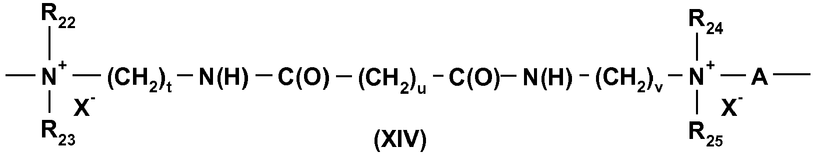 formule dans laquelle :
   R₂₂, R₂₃, R₂₄ et R₂₅, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₂₂, R₂₃, R₂₄ et R₂₅ ne représentent pas simultanément un atome d'hydrogène,
   t et u, identiques ou différents, sont des nombres entiers compris entre 1 et 6,
   v est égal à 0 ou à un nombre entier compris entre 1 et 34,
   X⁻, identique ou différente, désigne un anion tel qu'un halogènure,
   A désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(9) Les polymères quaternaires de vinylpyrrolidone et de vinylimidazole tels que par exemple les produits commercialisés sous les dénominations Luviquat® FC 905, FC 550 et FC 370 par la société B.A.S.F.
(10) Les polysaccharides cationiques notamment les celluloses et les gommes de galactomannanes cationiques.

Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.

Les dérivés d'éthers de cellulose comportant des groupements ammonium quaternaires décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.

Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Les protéines ou hydrolysats de protéines cationiques sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société MAYBROOOK et dénommés dans le dictionnaire CTFA "Steartrimonium Hydrolyzed Collagen" ;
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le "Croquat L" dont les groupements ammonium quaternaires comportent un groupement alkyle en C12 ;
- le "Croquat M" dont les groupements ammonium quaternairees comportent des groupements alkyle en C10-C18 ;
- le "Croquat S" dont les groupements ammonium quaternaires comportent un groupement alkyle en C18 ;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule (XV) : 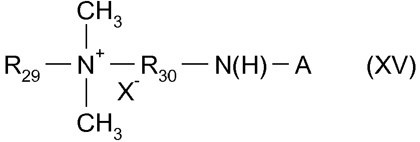 dans laquelle X⁻ est un anion d'un acide organique ou minéral, A désigne un reste de protéine dérivé d'hydrolysats de protéine de collagène, R₂₉ désigne un groupement lipophile comportant jusqu'à 30 atomes de carbone, R₃₀ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Parmi tous les polymères cationiques susceptibles d'être utilisés dans le cadre de la présente invention, on préfère mettre en oeuvre les composés des familles (1), (6), (7) et (8) et en particulier des familles (1), (6) et (7)
et leurs mélanges

Le ou les polymères cationiques ii) peuvent être présents en une teneur allant de 0,01 à 10 % en poids, mieux encore entre 0,1 eà 8 % en poids, et encore plus préférentiellement de 0,5 à 5 % en poids par rapport au poids total de la composition le ou les contenant.

De préférence, la composition de l'étape (3) comprend au moins un agent conditionneur cationique.

Selon un mode de réalisation particulier, la composition comprenant le ou les agents de traitement des cheveux, après un temps de pose en général inférieur à 20 min, de préférence allant de 1 à 15 minutes, est rincée avant mise en oeuvre de l'étape d'application de chaleur.

Les compositions mises en oeuvre dans le procédé de l'invention et notamment celle contenant l'acide glyoxylique et/ou un de ses dérivés peuvent en outre comprendre au moins un ingrédient cosmétique usuel, notamment choisi parmi les propulseurs; les huiles, les corps gras non liquides et notamment les esters en C₈-C₄₀, les acides en C₈-C₄₀; les tensioactifs, les filtres solaires; les agents hydratants; les agents antipelliculaires; les agents antioxydants; les agents chélatants; les agents nacrants et opacifiants; les agents plastifiants ou de coalescence; les charges; les silicones et en particulier les polydiméthylsiloxanes; les épaississants polymères ou non; les gélifiants; les émulsionnants; les polymères conditionneurs ou coiffants; les parfums; les agents d'alcalinisation ou d'acidification;les silanes ; les agents de réticulation. La composition peut bien évidemment comprendre plusieurs ingrédients cosmétiques figurant dans la liste ci-dessus.

Ces compositions peuvent en outre comprendre un ou plusieurs solvants organiques notamment hydrosolubles tels que les alcools en C₁-C₇; on peut notamment citer les monoalcools aliphatiques en C₁-C₇ ou aromatiques en C₆-C₇, les polyols et les éthers de polyols en C₃-C₇, qui peuvent être employés seuls ou en mélange avec de l'eau.

Selon leur nature et la destination des compositions, les ingrédients cosmétiques usuels peuvent être présents en des quantités usuelles, aisément déterminables par l'homme du métier, et qui peuvent être comprises, pour chaque ingrédient, entre 0,01 à 80% en poids. L'homme de métier veillera à choisir les ingrédients entrant dans la composition, ainsi que leurs quantités, de manière à ce qu'ils ne nuisent pas aux propriétés des compositions utiles dans la présente invention. Selon un mode de réalisation particulier, la composition contenant l'acide glyoxylique et/ou un de ses dérivés comprend au moins un polymère cellulosique non ionique.

Par polymère « *cellulosique* », on entend selon l'invention tout composé polysaccharidique possédant dans sa structure des enchaînements de résidus glucose unis par des liaisons β-1,4; Ainsi, les polymères cellulosiques de l'invention peuvent être choisis parmi les celluloses non substituées y compris sous une forme microcristalline et les éthers de cellulose. Parmi ces polymères cellulosiques, on distingue les éthers de celluloses, les esters de celluloses et les esters éthers de celluloses.

A titre d'exemple de polymères cellulosiques non ioniques, on peut citer les (C₁-C₄)alkylcelluloses telles que les méthylcelluloses et les éthylcelluloses (par exemple Ethocel standard 100 Premium de DOW CHEMICAL) ; les (poly)hydroxy(C₁-C₄)alkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON); les celluloses mixtes (poly)hydroxy(C₁-C₄)alkyl-(C₁-C₄)alkylcelluloses telles que les hydroxypropyl-méthylcelluloses (par exemple Methocel E4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutyl-méthylcelluloses.

Selon un mode de réalisation particulier, les polymères cellulosiques sont des polymères cellulosiques non ioniques et comprenant des chaines alkyles comportant de 1 à 6 atomes de carbone.. De préférence, le ou les polymères cellulosiques de l'invention sont choisis parmi les éthers de celluloses en particulier les celluloses hydroxyalkyl-alkyl celluloses telles que les (poly)hydroxy(C₁-C₄)alkyl-(C₁-C₄)alkylcelluloses en particulier les hydroxypropyl-méthylcelluloses (par exemple Methocel F4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutyl-méthylcelluloses.

Les polymères cellulosiques préférés sont des polymères cellulosiques qui ne comportent pas de chaîne grasse i.e. qui de préférence ne comportent pas de chaine comprenant plus de 10 atomes de carbone.

Le ou les polymères cellulosiques peuvent être présents dans la composition contenant l'acide glyoxylique et/ou un de ses dérivés dans des teneurs allant de 0,05% à 10 % en poids, en particulier de 0,1 % à 5 % en poids, et mieux encore de 0,1 % à 3% en poids, par rapport au poids total de la composition.

Après le rinçage de la composition appliquée à l'étape (ii), le procédé de l'invention comprend ensuite une phase d'application de chaleur comprenant au moins une étape de chauffage à une température d'au moins 150°C°C.
Selon un mode de réalisation, le chauffage à une température d'au moins 150°C est mis en oeuvre à partir d'un fer à lisser. Le lissage au fer est connu de l'état de la technique. Il consiste à lisser les cheveux avec une pince plate chauffante, généralement métallique. Les fers de lissage sont généralement utilisés à une température d'au moins 150°C, de préférence variant de 200 à 250°C.

Cette phase de lissage par application de chaleur peut être mise en oeuvre en deux étapes : une première étape qui consiste à lisser les cheveux au moyen d'une brosse et d'un sèche-cheveux (étape connue sous le nom de brushing). Cette étape de brushing est généralement mise en oeuvre entre 40 et 70°C ; une seconde étape qui consiste à lisser les cheveux avec un fer à lisser telle que décrite précédemment.

Selon un mode de réalisation particulier, le procédé de l'invention comprend l'application de la composition contenant l'acide glyoxylique et/ou un de ses dérivés sur cheveux secs, un temps de contact de la composition sur les cheveux allant de 10 et 60 minutes, de préférences de 20 à 40 minutes, l'application sans rinçage d'une composition comprenant au moins un composé choisi parmi les alcools gras, les polymères cationiques et les tensio-actifs cationiques, puis éventuellement après rinçage, le lissage des cheveux à la brosse et au sèche cheveux (brushing), suivi d'un lissage des cheveux au fer à lisser à une température d'au moins 150°C, de préférence variant de 200 à 250°C, de préférence 200 à 230°C.

Selon une variante d'un procédé de l'invention, l'application de la composition contenant de l'acide glyoxylique et/ou un de ses dérivés est réalisée sur cheveux préalablement shampooinés. Le shampoing mis en oeuvre dans le procédé de l'invention comprend de préférence d'au moins un tensio-actif amphotère associé éventuellement à au moins un tensioactif cationique. Selon un mode de réalisation particulier, le shampoing utilisé ne contient pas de tensioactifs anioniques.

Les tensioactifs amphotères qui peuvent être utilisés sont par exemple des tensioactifs bétaïniques tels que la cocobétaine ou la cocoamidopropylbétaïne ou les composés classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

Selon ce mode de réalisation, les cheveux sont rincés et de préférence pré-séchés (séchage total ou séchage partiel au sèche-cheveux ou avec une serviette) avant l'application de la composition comprenant l'acide glyoxylique et/ou au moins un de ses dérivés.

Selon une variante, le procédé de l'invention peut comprendre l'application d'autres agents capillaires en pré- ou post traitement des étapes (i) et (iii). De préférence, le procédé de l'invention comprend après le lavage des cheveux et avant l'application de la composition contenant de l'acide glyoxylique et/ou au moins un de ces dérivés un pré-traitement qui comprend l'application sur les cheveux, de préférence sur les cheveux mouillés d'une composition comprenant au moins un composé silicié comprenant au moins un atome de silicium tel qu'un alcoxysilane éventuellement organiquement modifiés notamment par un ou plusieurs groupes amino, une silicone par exemple un polydimethylsiloxane éventuellement substitué notamment par un ou plusieurs groupes amino. A titre d'exemple de composés siliciés, on peut citer l'aminopropyltriéthyloxysilane, les amodimethicones. L'application de ce prétraitement n'est de préférence pas rincée avant l'application de la composition contenant de l'acide glyoxylique et/ou au moins un de ces dérivés et de préférence est suivie d'un pré séchage (séchage total ou séchage partiel au sèche cheveux ou avec une serviette) avant l'application de la composition contenant de l'acide glyoxylique et/ou au moins un de ces dérivés.

Selon un mode de réalisation particulier, le lissage au fer à lisser s'effectue en plusieurs passages sur les cheveux, en général 8 à 10 passages.

### Exemple

On applique sur des cheveux naturellement frisés ayant été préalablement lavés rincés puis pré-séchés, la composition à l'acide glyoxylique décrite ci-dessous, les teneurs sont indiquées en matière première brute (produit commercial en l'état)

| **Nom chimique** | **Teneur (g)** |
|---|---|
| ACIDE LACTIQUE | 2,5 |
| ACIDE GLYOXYLIQUE EN SOLUTION AQUEUSE A 50% | 16 |
| HYDROXYPROPYL METHYL CELLULOSE (**METHOCEL F 4 M de DOW CHEMICAL**) | 1 |
| HOMOPOLYMERE DE CHLORURE DE METHACRYLATE D'ETHYL TRI-METHYL AMMONIUM RETICULE, EN DISPERSION DANS UN MEL. D'ESTERS A 50 % **(SALCARE SC 96 de BASF)** | 2,5 |
| AMINO MODIFIED SILICONE - POLYETHER COPOLYMER (**SILSOFT A+ de MOMENTIVE PERFORMANCE MATERIAL S)** | 2 |
| N-COCOYL AMIDOETHYL, N-ETHOXYCARBOXYMETHYL GLYCINATE DE SODIUM EN SOLUTION AQUEUSE A 31.5% **(MIRANOL C2M CONC NP de RHODIA)** | 2 |
| HYDROXYDE DE SODIUM | qs pH 2.2 |
| EAU DESIONISEE | Qsp 100 |

Sans rinçage de cette composition à l'acide glyoxylique et après un temps de pose de 20 min, on applique la composition suivante :

| **Nom chimique** | **Teneur (g)** |
|---|---|
| ACIDE LACTIQUE | 0,1 |
| ACIDE CITRIQUE | 0,3 |
| HUILE DE VASELINE (**MARCOL 82 de EXXONMOBIL CHEMICAL)** | 3 |
| ALCOOL CETYLSTEARYLIQUE (C16/C18 50/50) (**NAFOL 1618 EN de SASOL**) | 9 |
| MELANGE MYRISTATE/PALMITATE/STEARATE DE MYRISTYLE/CETYLE/STEARYLE (**CRODAMOL MS- PA-(MH) de CRODA**) | 1 |
| CHLORURE DE CETYL TRIMETHYL AMMONIUM EN SOLUTION AQUEUSE (**GENAMIN CTAC 25 de CLARIANT**) | 3,2 |
| METHOSULFATE DE DIPALMITOYLETHYL HYDROXYETHYL METHYL AMMONIUM (30)/ ALCOOL CETEARYLIQUE(70) (**DEHYQUART F 30 de COGNIS**) | 4,5 |
| Conservateurs | QS |

Après un temps de pause de 10 min, on rince les cheveux, on réalise un brushing puis on lisse au fer à lisser. On obtient ainsi des cheveux défrisés, lisses avec un bon toucher cosmétique et sans inconvénients annexes liés à l'application.

### Exemple 2.

On obtient des résultats améliorés en utilisant en prétraitement sur les cheveux initialement lavés un mélange 50/50 des deux compositions A et B ci-dessous contenant en tant que composé silicié le 3-aminopropyltriethoxysilane puis en appliquant sans rinçage mais avec préséchage le process tel que décrit dans l'exemple 1 à partir de l'application de la composition avec acide glyoxylique.

| | **Composition A (en g)** | **Composition B (en g)** | |
|---|---|---|---|
| HYDROXYETHYL CELLULOSE (PM : 1.300.000) (**NATROSOL 250 HHR PC de ASHLAND**) | 1 | - | |
| MELANGE POLY DIMETHYLSILOXANE ALPHA-OMEGA DIHYDROXYLE / CYCLOPENTA DIMETHYLSILOXANE (14.7/85.3) (**XIAMETER PMX-1501 FLUID de DOW CORNING**) | - | 65 | |
| 3-AMINOPROPYLTRIETHOXYSILANE (**SILSOFT A-1100 de MOMENTIVE PERFORMANCE MATERIALS**) | 1 | | |
| ALCOOL ETHYLIQUE ABSOLU | | 5 | |
| CONSERVATEURS | Qs | - | |
| HUILE DE RICIN AVEC 40 OE (**CREMOPHOR CO 40 SURFACTANT de BASF)** | 2,4 | - | |
| ACIDE LACTIQUE | 0,25 | - | |
| EAU DEIONISEE | QSP 100 | - | |
| CYCLOPENTA DIMETHYLSILOXANE | | QSP 100 | |

## Revendications

1. Procédé de lissage/défrisage des fibres kératiniques, en particulier des cheveux, qui comprend
(i) l'application sur lesdites fibres, d'une composition cosmétique comprenant au moins de l'acide glyoxylique et/ou au moins un de ses dérivés choisis parmi les esters d'acide glyoxylique, les amides d'acide glyoxyliques, les acétals et hémiacétals d'acide glyoxylique, la composition présentant un pH inférieur ou égal à 4,
(ii) un temps de pose de la composition à l'acide glyoxylique et/ou au moins un de ses dérivés choisis parmi les esters d'acide glyoxylique, les amides d'acide glyoxyliques, les acétals et hémiacétals d'acide glyoxylique d'au moins 10 min, suivi de
(iii) l'application sans rinçage d'une composition comprenant au moins un composé de traitement des cheveux choisi parmi les alcools gras aliphatiques à longue chaîne comprenant de 8 à 40 atomes de carbone et comprenant au moins un groupe hydroxyle OH qui ne sont ni oxyalkylénés, ni glycérolés, puis
(iv) une phase d'application de chaleur comprenant au moins une étape de chauffage à une température d'au moins 150°C.

2. Procédé selon la revendication 1, dans lequel l'acide glyoxylique et/ou ses dérivés se présente sous forme libre ou sous forme d'hydrate ou sous forme de sels.

3. Procédé selon la revendication 1, dans lequel la composition de l'étape (i) comprend de l'acide glyoxylique sous forme libre ou sous forme d'hydrate.

4. Procédé selon l'une quelconque des revendications précédentes dans laquelle la quantité d'acide glyoxylique et/ou de ses dérivés est d'au moins 3 % en poids du poids total de la composition, de préférence allant de 3 à 10 % en poids du poids total de la composition.

5. Procédé selon l'une quelconque des revendications précédentes dans lequel la composition contenant l'acide glyoxylique et/ou au moins un de ses dérivés comprend de plus un polymère cellulosique non ionique.

6. Procédé selon l'une quelconque des revendications précédentes dans lequel dans l'étape (ii) le temps de pose varie de 10 à 60 min, de préférence de 20 à 35 min.

7. Procédé selon l'une quelconque des revendications précédentes dans lequel à l'étape (iii) on applique en outre au moins un composé de traitement des cheveux choisi parmi les polymères cationiques et/ou les tensio-actifs cationiques.

8. Procédé selon la revendication 7 dans lequel le composé conditionneur choisi parmi les alcools gras et/ou les polymères cationiques et/ou les tensio-actifs cationiques est compris dans une composition exempte d'acide glyoxylique et/ou un de ses dérivés.

9. Procédé selon l'une quelconque des revendications précédentes dans lequel le ou les composés de traitement des cheveux choisi parmi les alcools gras et/ou les agents conditionneurs cationiques sont présents pour chacune des catégories en une quantité allant de 0,01 à 10 % en poids, mieux encore entre 0,1 à 8 % en poids, et encore plus préférentiellement de 0,5 à 5% en poids par rapport au poids total de la composition le ou les contenant.

10. Procédé selon l'une quelconque des revendications précédentes dans lequel la composition (iii) comprenant le ou les composés de traitement des cheveux choisis parmi les alcools gras et/ou les agents conditionneurs cationiques, après un temps de pose en général inférieur à 20 min, de préférence allant de 1 à 15 minutes, est rincée avant mise en oeuvre de l'étape de chauffage.

11. Procédé selon l'une quelconque des revendications précédentes dans lequel la phase d'application de chaleur comprend une seule étape qui consiste à lisser les cheveux avec un fer à lisser à une température d'au moins 150°C, de préférence de 200 à 250°C.

12. Procédé selon l'une quelconque des revendications précédentes dans lequel la phase d'application de chaleur comprend une première étape qui consiste à lisser les cheveux au moyen d'une brosse et d'un sèche-cheveux (étape connue sous le nom de brushing) et une seconde étape qui consiste à lisser les cheveux avec un fer à lisser à une température d'au moins 150°C de préférence de 200 à 250°C.

13. Procédé selon l'une quelconque des revendications précédentes comprenant avant l'application de la composition de l'étape (i) un pré-traitement qui comprend l'application d'une composition comprenant au moins un composé silicié comprenant au moins un atome de silicium, de préférence un alcoxysilane éventuellement organiquement modifié par un ou plusieurs groupes amino, une silicone de préférence un polydimethylsiloxane éventuellement substitué par un ou plusieurs groupes amino.

14. Procédé selon la revendication précédente dans lequel la composition de prétraitement comprenant au moins un composé silicié n'est pas rincée.

## Patentansprüche

1. Verfahren zum Glätten/Entkrausen von Keratinfasern, insbesondere Haar, das umfasst:
(i) das Aufbringen auf die Fasern, einer kosmetischen Zusammensetzung, umfassend mindestens Glyoxylsäure und/oder mindestens ein Derivat davon, ausgewählt aus Glyoxylsäureestern, Glyoxylsäureamiden, Glyoxylsäureacetalen und -halbacetalen, wobei die Zusammensetzung einen pH-Wert kleiner oder gleich 4 aufweist,
(ii) eine Einwirkzeit der Zusammensetzung aus Glyoxylsäure und/oder mindestens einem Derivat davon, ausgewählt aus Glyoxylsäureestern, Glyoxylsäureamiden, Glyoxylsäureacetalen und -halbacetalen von mindestens 10 Min., gefolgt vom
(iii) Aufbringen ohne Ausspülen einer Zusammensetzung, umfassend mindestens eine Verbindung zur Haarbehandlung, ausgewählt aus langkettigen aliphatischen Fettalkoholen, umfassend 8 bis 40 Kohlenstoffatome und umfassend mindestens eine Hydroxylgruppe OH, die weder oxyalkyliert noch glyceriniert sind, dann
(iv) eine Phase zum Aufbringen von Wärme, umfassend mindestens einen Schritt zum Erwärmen auf eine Temperatur von mindestens 150 °C.

2. Verfahren nach Anspruch 1, wobei die Glyoxylsäure und/oder ein Derivate davon in freier Form oder in Hydratform oder in Salzform vorliegt/vorliegen.

3. Verfahren nach Anspruch 1, wobei die Zusammensetzung aus Schritt (i) Glyoxylsäure in freier Form oder in Hydratform umfasst.

4. Verfahren nach einem der vorhergehenden Ansprüche, wobei die Menge an Glyoxylsäure und/oder einem Derivat davon mindestens 3 Gew.-% des Gesamtgewichts der Zusammensetzung beträgt, vorzugsweise im Bereich zwischen 3 bis 10 Gew.-% des Gesamtgewichts der Zusammensetzung liegt.

5. Verfahren nach einem der vorhergehenden Ansprüche, wobei die Zusammensetzung, die Glyoxylsäure und/oder ein mindestens ein Derivat davon enthält, außerdem ein nichtionisches Polymer auf Cellulosebasis umfasst.

6. Verfahren nach einem der vorhergehenden Ansprüche, wobei in Schritt (ii) die Einwirkzeit von 10 bis 60 Min., vorzugsweise von 20 bis 35 Min. reicht.

7. Verfahren nach einem der vorhergehenden Ansprüche, wobei in Schritt (iii) mindestens eine Haarbehandlungsverbindung, ausgewählt aus Fettalkoholen und/oder kationischen Polymeren und/oder kationischen Tensiden, aufgebracht wird.

8. Verfahren nach Anspruch 7, wobei die Konditionierverbindung, die aus Fettalkoholen und/oder kationischen Polymeren und/oder kationischen Tensiden ausgewählt ist, in einer Zusammensetzung enthalten ist, die frei ist von Glyoxylsäure und/oder einem Derivat davon.

9. Verfahren nach einem der vorhergehenden Ansprüche, wobei die Haarbehandlungsverbindung(en), ausgewählt aus Fettalkoholen und/oder kationischen Konditioniermitteln, für jede der Kategorien in einer Menge im Bereich von 0,01 bis 10 Gew.-%, besser 0,1 bis 8 Gew.-%, und noch bevorzugter 0,5 bis 5 Gew.-%, bezogen auf das Gesamtgewicht der Zusammensetzung, die sie enthält, vorhanden ist/sind.

10. Verfahren nach einem der vorhergehenden Ansprüche, wobei die Zusammensetzung (iii), umfassend die Haarbehandlungsverbindung(en), ausgewählt aus Fettalkoholen und/oder kationischen Konditioniermitteln, nach einer Einwirkzeit von im Allgemeinen weniger als 20 Min., vorzugsweise im Bereich von 1 bis 15 Minuten, vor dem Schritt zum Aufbringen von Wärme ausgespült wird.

11. Verfahren nach einem der vorhergehenden Ansprüche, wobei der Schritt zum Aufbringen von Wärme einen einzigen Schritt umfasst, der darin besteht, das Haar mit einem Glätteisen bei einer Temperatur von mindestens 150 °C, vorzugsweise von 200 bis 250 °C, zu glätten.

12. Verfahren nach einem der vorhergehenden Ansprüche, wobei die Phase zum Aufbringen von Wärme einen ersten Schritt umfasst, der darin besteht, das Haar mittels einer Bürste und eines Föns zu glätten (ein Schritt, der unter dem Namen Brushing bekannt ist) und einen zweiten Schritt, der darin besteht, das Haar mit einem Glätteisen bei einer Temperatur von mindestens 150 °C, vorzugsweise von 200 bis 250 °C, zu glätten.

13. Verfahren nach einem der vorhergehenden Ansprüche, umfassend vor dem Aufbringen der Zusammensetzung von Schritt (i) eine Vorbehandlung, die das Aufbringen einer Zusammensetzung umfasst, umfassend mindestens eine siliziumhaltige Verbindung, umfassend mindestens ein Siliziumatom, vorzugsweise ein Alkoxysilan, das gegebenenfalls organisch mit einer oder mehreren Aminogruppen modifiziert ist, ein Silikon, vorzugsweise ein Polydimethylsiloxan, das gegebenenfalls mit einer oder mehreren Aminogruppen substituiert ist.

14. Verfahren nach dem vorhergehenden Anspruch, wobei die Vorbehandlungszusammensetzung, die mindestens eine siliziumhaltige Verbindung enthält, nicht ausgespült wird.

## Claims

1. Method for straightening/relaxing keratin fibers, in particular the hair, which comprises
(i) the application to said fibers of a cosmetic composition comprising at least glyoxylic acid and/or at least one derivative thereof chosen from glyoxylic acid esters, glyoxylic acid amides and glyoxylic acid acetals and hemiacetals, the composition having a pH of less than or equal to 4,
(ii) a leave-on time for the composition comprising glyoxylic acid and/or at least one derivative thereof chosen from glyoxylic acid esters, glyoxylic acid amides and glyoxylic acid acetals and hemiacetals of at least 10 min, followed by
(iii) the application, without rinsing, of a composition comprising at least one hair treatment compound chosen from long-chain aliphatic fatty alcohols comprising from 8 to 40 carbon atoms and comprising at least one hydroxyl group OH which are neither oxyalkylenated nor glycerolated, and then by
(iv) a heat application step comprising at least one step of heating at a temperature of at least 150°C.

2. Method according to Claim 1, wherein the glyoxylic acid and/or derivatives thereof is in free form or in hydrate form or in salt form.

3. Method according to Claim 1, wherein the composition of step (i) comprises glyoxylic acid in free form or in hydrate form.

4. Method according to any one of the preceding claims, wherein the amount of glyoxylic acid and/or of derivatives thereof is at least 3% by weight of the total weight of the composition, preferably ranging from 3% to 10% by weight of the total weight of the composition.

5. Method according to any one of the preceding claims, wherein the composition containing glyoxylic acid and/or a derivative thereof additionally comprises a nonionic cellulose-based polymer.

6. Method according to any one of the preceding claims, wherein, in step (ii), the leave-on time ranges from 10 to 60 min, preferably from 20 to 35 min.

7. Method according to any one of the preceding claims, wherein, in step (iii), at least one hair treatment compound chosen from cationic polymers and/or cationic surfactants is also applied.

8. Method according to Claim 7, wherein the conditioning compound chosen from fatty alcohols and/or cationic polymers and/or cationic surfactants is included in a composition free of glyoxylic acid and/or a derivative thereof.

9. Method according to any one of the preceding claims, wherein the hair treatment compound(s) chosen from fatty alcohols and/or cationic conditioning agents is (are) present for each of the categories in an amount ranging from 0.01% to 10% by weight, even better still from 0.1% to 8% by weight and even more preferentially from 0.5% to 5% by weight relative to the total weight of the composition containing it or them.

10. Method according to any one of the preceding claims, wherein the composition (iii) containing the hair treatment compound(s) chosen from fatty alcohols and/or cationic conditioning agents, after a leave-on time of generally less than 20 min, preferably ranging from 1 to 15 min, is rinsed off before the heat application step is carried out.

11. Method according to any one of the preceding claims, wherein the heat application step comprises a single step which consists in straightening the hair with a straightening iron at a temperature of at least 150°C, preferably from 200 to 250°C.

12. Method according to any one of the preceding claims, wherein the heat application step comprises a first step which consists in straightening the hair by means of a brush and a hairdryer (step known as blow drying) and a second step which consists in straightening the hair with a straightening iron at a temperature of at least 150°C, preferably from 200 to 250°C.

13. Method according to any one of the preceding claims, comprising, before the application of the composition of step (i), a pretreatment which comprises the application of a composition comprising at least one siliceous compound comprising at least one silicon atom, preferably an alkoxysilane optionally organically modified with one or more amino groups, or a silicone, preferably a polydimethylsiloxane optionally substituted with one or more amino groups.

14. Method according to the preceding claim, wherein the pretreatment composition comprising at least one siliceous compound is not rinsed off.
